# EUROPEAN PATENT APPLICATION

(11) **EP 1 440 980 A2**
(43) Date of publication of application: **28.07.2004**
(21) Application number: 03028981.3
(22) Date of filing: 16.11.1995
(51) Int. Cl.: C07K 14/47, A61K 38/17, A61P 25/02

(54) **Peptide analogs of human myelin basic protein**

(30) Priority: 18.11.1994 US 342408
(62) Divisional of application: 95942843.4
(71) Applicant: NEUROCRINE BIOSCIENCES, INC., San Diego, CA 92121-1102 (US)
(72) Inventor: Ling, Nicholas, San Diego, CA 92121 (US); Gaur, Amitabh, San Diego, CA 92129 (US); Conlon, Paul J., Solana Beach, CA 92075 (US); Steinman, Lawrence, Palo Alto, CA 94301 (US)
(74) Representative: Gowshall, Jonathan Vallance

(57) **Abstract**

The present invention is directed toward peptide analogs of human myelin basic protein. The peptide analog is at least seven amino acids long and derived from residues 83 to 99 of human myelin basic protein. The analogs are altered from the native sequence at least at positions 91, 95, or 97. Additional alterations may be made at other positions. Pharmaceutical compositions containing these peptide analogs are provided. The peptide analogs are useful for treating multiple sclerosis.

## Description

### Technical Field

The present invention relates generally to methods for treating multiple sclerosis by using peptide analogs of human myelin basic protein.

### Background of the Invention

Multiple sclerosis (MS) is a chronic, inflammatory disease that affects approximately 250,000 individuals in the United States. Although the clinical course may be quite variable, the most common form is manifested by relapsing neurological deficits, in particular, paralysis, sensory deficits, and visual problems.

The inflammatory process occurs primarily within the white matter of the central nervous system and is mediated by T lymphocytes, B lymphocytes, and macrophages. These cells are responsible for the demyelination of axons. The characteristic lesion in MS is called the plaque due to its macroscopic appearance.

Multiple sclerosis is thought to arise from pathogenic T cells that somehow evaded mechanisms establishing self-tolerance, and attack normal tissue. T cell reactivity to myelin basic protein may be a critical component in the development of MS. The pathogenic T cells found in lesions have restricted heterogeneity of antigen receptors (TCR). The T cells isolated from plaques show rearrangement of a restricted number of Vα and Vβ gene segments. In addition, the TCRs display several dominant amino acid motifs in the third complementarity determining region (CDR), which is the major antigen contact site. All together, three CDR3 motifs have been identified in T cell clones known to recognize an epitope within amino acids 82-106 of myelin basic protein. These motifs were found in 44% of rearranged TCR sequences involving one particular Vβ gene rearranged in T cells isolated from brain of two patients with MS.

A definitive treatment for MS has not been established. Historically, corticosteroids and ACTH have been used to treat MS. Basically, these drugs reduce the inflammatory response by toxicity to lymphocytes. Recovery may be hastened from acute exacerbations, but these drugs do not prevent future attacks or prevent development of additional disabilities or chronic progression of MS (Carter and Rodriguez, *Mayo Clinic Proc.* 64:664, 1989; Weiner and Hafler, *Ann. Neurol. 23*:211, 1988). In addition, the substantial side effects of steroid treatments make these drugs undesirable for long-term use.

Other toxic compounds, such as azathioprine, a purine antagonist, cyclophosphamide, and cyclosporine have been used to treat symptoms of MS. Like corticosteroid treatment, these drugs are beneficial at most for a short term and are highly toxic. Side effects include increased malignancies, leukopenias, toxic hepatitis, gastrointestinal problems, hypertension, and nephrotoxicity (Mitchell, *Cont. Clin. Neurol.* 77:231, 1993; Weiner and Hafler, *supra*). Antibody based therapies directed toward T cells, such as anti-CD4 antibodies, are currently under study for treatment of MS. However, these agents may cause deleterious side effects by immunocompromising the patient.

More recently, cytokines such as IFN-γ and IFN-β have been administered in attempts to alleviate the symptoms of MS. However, a pilot study involving IFN-γ was terminated because 7 of 18 patients treated with this drug experienced a clinical exacerbation within one month after initiation of treatment. Moreover, there was an increase in the specific response to MBP (Weiner and Hafler, *supra*).

Betaseron, a modified beta interferon, has recently been approved for use in MS patients. Although Betaseron treatment showed some improvement in exacerbation rates (Paty et al., *Neurology 43*:662, 1993), there was no difference in the rate of clinical deterioration between treated and control groups (IFNB MS Study Group, *Neurology 43*:655, 1993; Paty et al., *supra*). Side effects were commonly observed. The most frequent of such side effects were fever (40%-58% of patients), flu-like symptoms (76% of patients), chills (46% of patients), mylagias (41% of patients), and sweating (23% of patients). In addition, injection site reactions (85%), including inflammation, pain, hypersensitivity and necrosis, were common (IFNB MS Study Group, *supra;* Connelly, *Annals of Pharm. 28*:610, 1994).

In view of the problems associated with existing treatments of MS, there is a compelling need for improved treatments which are more effective and are not associated with such disadvantages. The present invention exploits the use of peptide analogs which antagonize a T cell response to human myelin basic protein to effectively treat MS, while providing other related advantages.

### Summary of the Invention

The present invention provides peptide analogs comprising at least 7 amino acids selected from residues 83 to 99 of human myelin basic protein in which either L-lysine at position 91, L-threonine at position 95, or L-arginine at position 97 is altered to another amino acid. In one embodiment, the peptide analog comprises at least 7 amino acids selected from residues 86-99, L-lysine at position 91 is altered and one to three additional L-amino acids selected from residues 86, 87, 88, 95, 98 or 99 are altered to another amino acid. In a second related embodiment, L-threonine at position 95 is altered and one to three additional amino acids selected from residues 86, 87, 88, 91, 98 and 99 or 86, 87, 88, 97, 98, and 99 are altered to another amino acid. In a third related embodiment, L-arginine at position 97 is altered and one to three additional amino acids selected from residues 86, 87, 88, 95, 98 or 99 are altered to another amino acid.

Within another set of embodiments, the peptide analog comprises residues 83-99 of human myelin basic protein, wherein the peptide analogs preferably contain two to five alterations. In preferred aspects of the invention, the peptide analogs have altered residues 89, 91, 95 or 97 to alanine and the additional amino acids are altered to the corresponding D-form amino acid.

In other embodiments, peptide analogs comprise at least seven amino acids selected from residues 86 to 99 of human myelin basic protein in which either L-lysine at position 91, L-threonine at position 95, or L-arginine at position 97 is altered to another amino acid, and in addition the N-terminal and C-terminal amino acids are altered in order to reduce proteolysis upon administration of the peptide analog. In a preferred aspect, the N- and/or C-terminal amino acids are of the D-form.

In other embodiments, the peptide analogs comprise at least seven amino acids selected from residues 86 to 99 of human myelin basic protein in which either L-lysine at position 91, L-threonine at position 95, or L-arginine at position 97 is altered to another amino acid and in addition up to three other amino acid alterations are made. Any residue within 86-99 may be altered except that in a peptide analog in which residue 91 is altered, residue 97 may not be altered. Likewise, in a peptide analog in which residue 97 is altered, residue 91 may not be altered.

Other embodiments provide peptide analogs comprising at least seven amino acids selected from residues 86 to 99 of human myelin basic protein in which either L-lysine at position 91, L-threonine at position 95, or L-arginine at position 97 is altered to another amino acid. In preferred aspects, residue 91, 95 or 97 are altered to either alanine or the corresponding D-amino acid.

Further aspects of the present invention provide a pharmaceutical composition comprising a peptide analog according to the embodiments set out above in which the peptide analog is contained in a physiologically acceptable carrier or diluent.

Still additional aspects of the present invention provide methods of treating multiple sclerosis by administering to a patient a therapeutically effective amount of a pharmaceutical composition comprising a peptide analog as described above in combination with a physiologically acceptable carrier or diluent

These and other aspects of the invention will become evident upon reference to the following detailed description and attached drawings. In addition, various references are set forth below which describe in more detail certain procedures or compositions. Each of these references are incorporated herein by reference in their entirety as if each were individually noted for incorporation.

### Brief Description of the Drawings

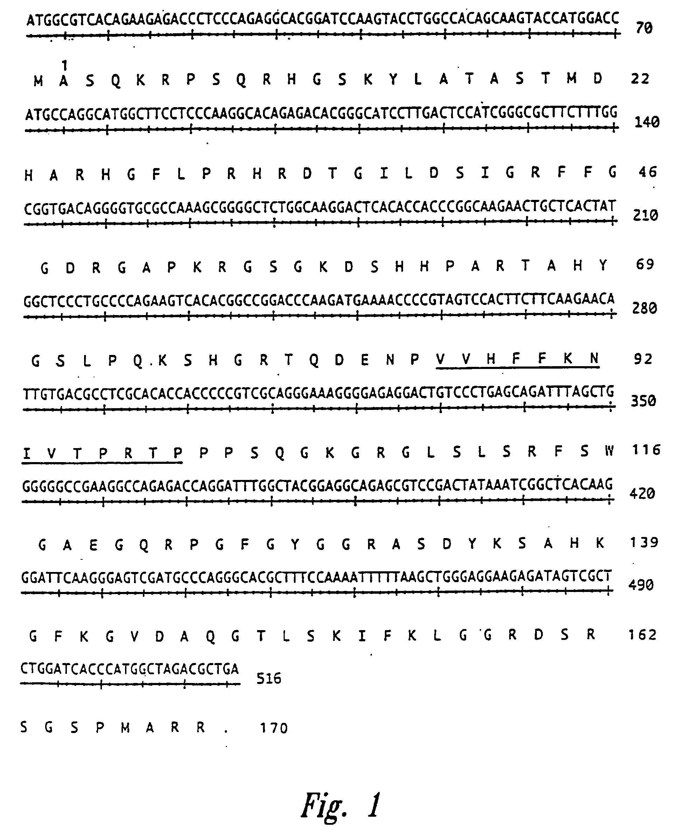

Figure 1 depicts DNA and predicted amino acid sequence for human myelin basic protein.

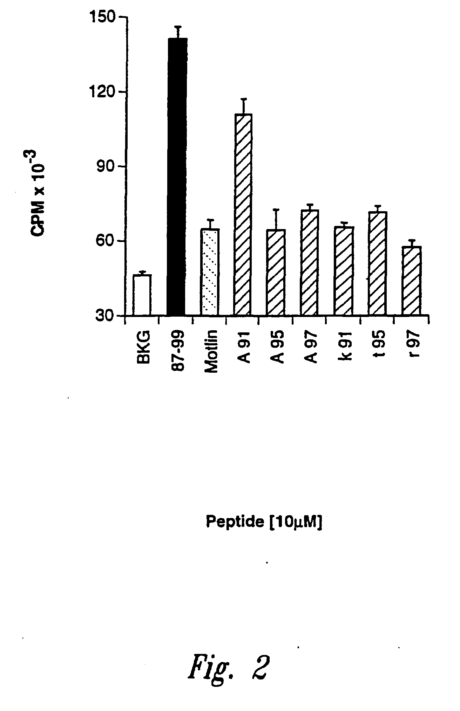

Figure 2 depicts the response of draining lymph node cells from Lewis rats immunized 9-10 days previously with MBP (87-99) to 10 µM of MBP (87-99), medium, the unrelated peptide motilin, and six different MBP analogs. A, L-alanine; k, D-Iysine; t, D-threonine; r, D-arginine.

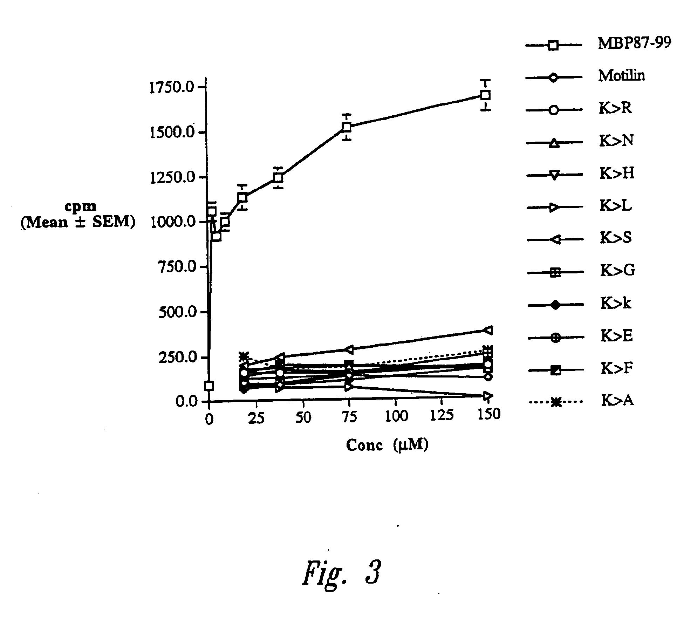

Figure 3 is a graph displaying the proliferative response of the T cell line NBI to residue 91-substituted analogs of human myelin basic protein (87-99). Ten different substitutions were tested. The proliferative response of the rat T cell line in response to concentrations of peptide analogs ranging from 0 to 150 µM was determined. The extent of proliferation is shown as counts per minute; standard errors of the mean were less than ±10%. MOT, motilin, a peptide unrelated to MBP; MBP (87-99), human myelin basic protein residues 87-99; K, lysine; R, arginine; N, asparagine; H, histidine; L, leucine; S, serine; G, glycine; k, D-Lysine; E, glutamic acid; F, phenylalanine; A, alanine.

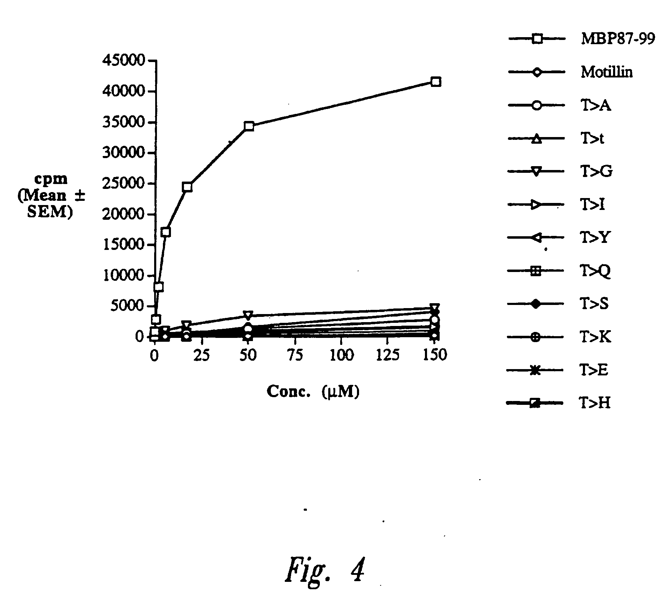

Figure 4 is a graph displaying the proliferative response of the T cell line NBI to residue 95-substituted analogs of human myelin basic protein (87-99). Ten different substitutions were tested. The proliferative response of the rat T cell line in response to concentrations of peptide analogs ranging from 0 to 150 µM was determined. The extent of proliferation is shown as counts per minute; standard errors of the mean were less than ±10%. MOT, motilin, a peptide unrelated to MBP; MBP (87-99), human myelin basic protein residues 87-99; T, threonine; A, alanine; t, D-threonine; G, glycine; I, isoleucine; Y, tyrosine; Q, glutamine; S, serine; K, lysine; E, glutamic acid; H, histidine.

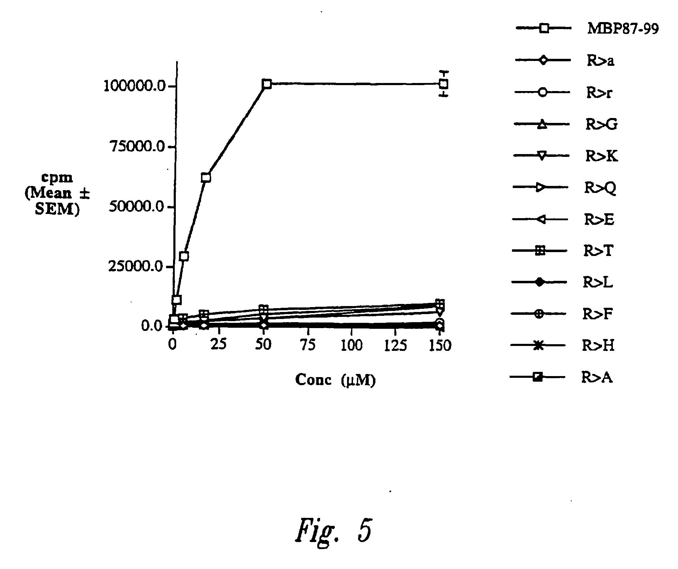

Figure 5 is a graph displaying the proliferative response of the T cell line NBI to residue 97-substituted analogs of human myelin basic protein. Eleven different substitutions were tested. The proliferative response of the T cells to concentrations of peptide analogs ranging from 0 to 150 µM was determined. The extent of proliferation is displayed as counts per minute. MBP 87-99, myelin basic protein (87-99); R, arginine; a, D-alanine; r, D-arginine; G, glycine; K, lysine; Q, glutamine; E, glutamic acid; T, threonine; L, leucine; F, phenylalanine; H, histidine; A, alanine.

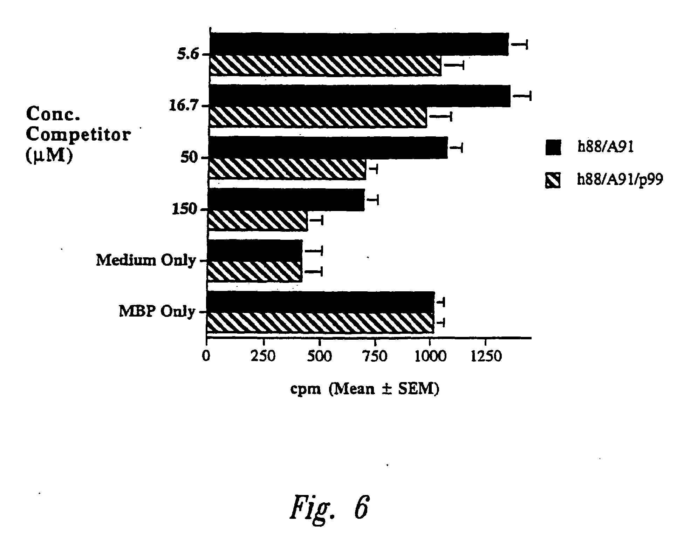

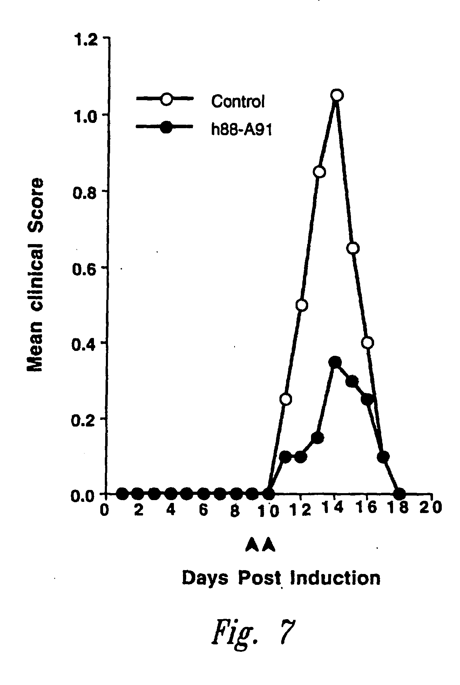

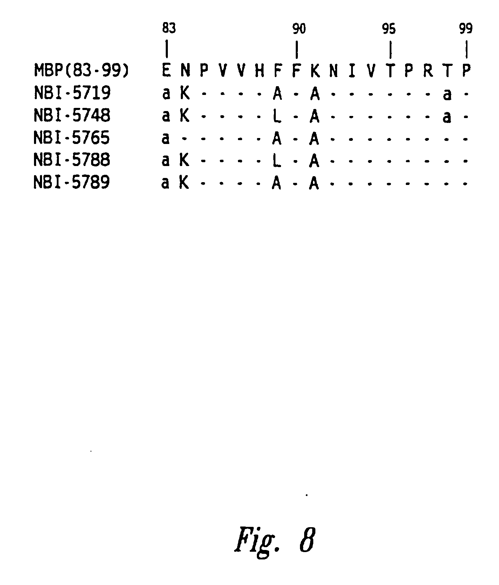

Figure 6 is a graph illustrating the ability of peptide analogs of MBP to inhibit proliferation of rat T cells that are reactive to MBP. The proliferative response of draining lymph node cells from rats immunized with MBP (87-99) to 16.7, 50, or 150 µM of each analog, or 5 µM of MBP (87-99) is displayed. Analogs were added in the presence of 5 µM MBP (87-99). The extent of proliferation is shown as counts per minute. Controls consisted of MBP (87-99) only at 5 µM and medium only. h88/A91 refers to a representative peptide analog of MBP (87-99) with D-histidine at residue 88 and alanine at residue 91; h88/A91/p99 refers to another representative peptide analog of MBP (87-99) with D-histidine at 88, alanine at residue 91, and D-proline at residue 99.

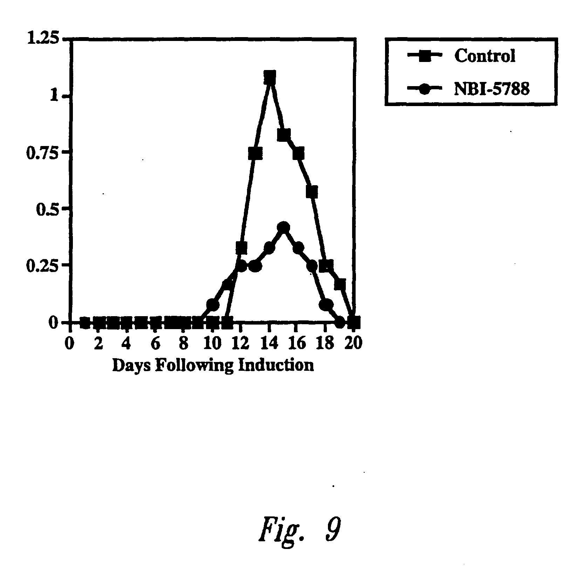

Figure 7 is a graph demonstrating the inhibition of EAE induction in Lewis rats following injection of MBP (87-99). Arrows indicate days that either PBS (control) or h88/A91 peptide analog were administered. EAE was recorded as 0, no symptoms; 1, tail paralysis; 2, hind limb weakness; 3, hind limb paralysis; 4, hind and front limb paralysis.
Figure 8 depicts the amino acid sequence in single letter code for residues 83 to 99 of human myelin basic protein and the amino acid sequences of the peptide analogs NBI-5719, NBI-5748, NBI-5765, NBI-5788, and NBI-5789. A dash indicates amino acid identity. MBP (83-99), myelin basic protein residues 83 to 99; a, D-alanine; A, L-alanine; K, L-lysine; L, L-leucine.
Figure 9 is a graph demonstrating the inhibition of EAE induction in Lewis rats following injection of MBP (83-99). Lewis rats were injected with MBP (83-99) at day 0. At day 9, rats were injected with either a control peptide, sperm whale myoglobin (110-121) or the peptide analog, NBI-5788. Each data point represents the average of the clinical score of six animals.

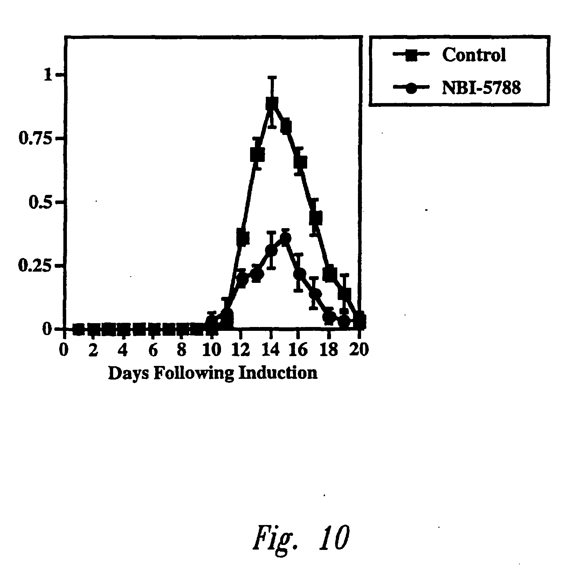

Figure 10 is a graph demonstrating the inhibition of EAE induction in Lewis rats following injection of MBP (83-99). Lewis rats were injected with MBP (83-99) at day 0. At day 9, rats were injected with either a control peptide, sperm whale myoglobin (110-121) or the peptide analog, NBI-5788. Each data point represents the average of the clinical score of six animals.

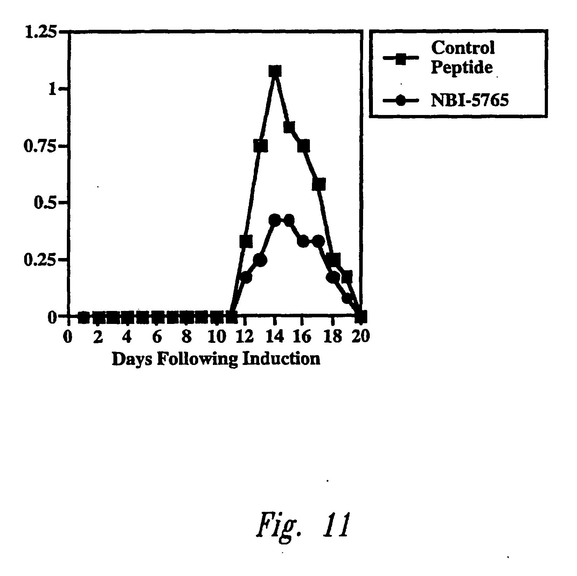

Figure 11 is a graph demonstrating the inhibition of EAE induction in Lewis rats following injection of MBP (83-99). Lewis rats were injected with MBP (83-99) at day 0. At day 9, rats were injected with either a control peptide, sperm whale myoglobin (110-121) or the peptide analog, NBI-5765. Each data point represents the average of the clinical score of six animals.

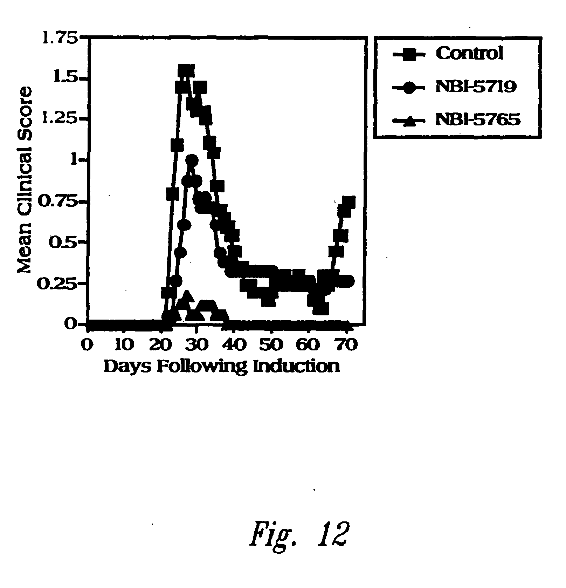

Figure 12 is a graph demonstrating the inhibition of EAE induction in SJL/J mice following injection of MBP (87-99). Groups of mice were injected intraperitoneally on a weekly basis for four weeks with either a control peptide or the peptide analog, NBI-5719 or NBI-5765. Each data point represents the average of the clinical score for ten mice.

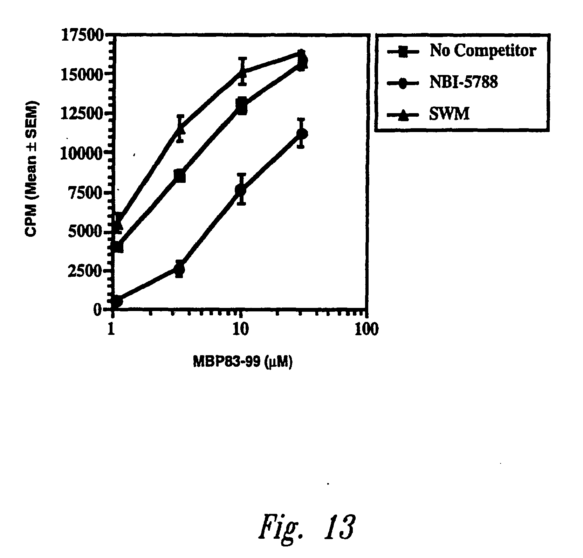

Figure 13 is a graph illustrating the ability of a peptide analog of MBP to inhibit proliferation of a Dr2a restricted human T cell clone, which is reactive to MBP. The proliferative response of the T cell clone incubated with varying concentrations of MBP (83-99) and 50 micromolar of either the peptide analog or sperm whale myoglobin, the control peptide, is depicted.

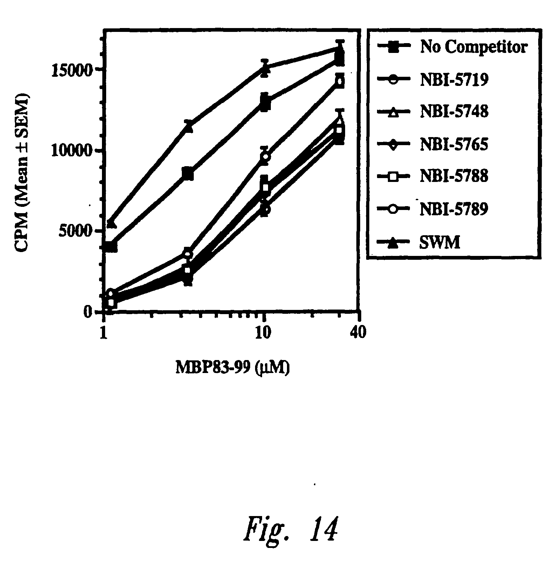

Figure 14 is a graph illustrating the ability of peptide analogs of MBP to inhibit proliferation of a Dr2a restricted human T cell clone, which is reactive to MBP. The proliferative response of the T cell clone incubated with varying concentrations of MBP (83-99) and 50 micromolar of either the peptide analog or sperm whale myoglobin, the control peptide, is depicted.

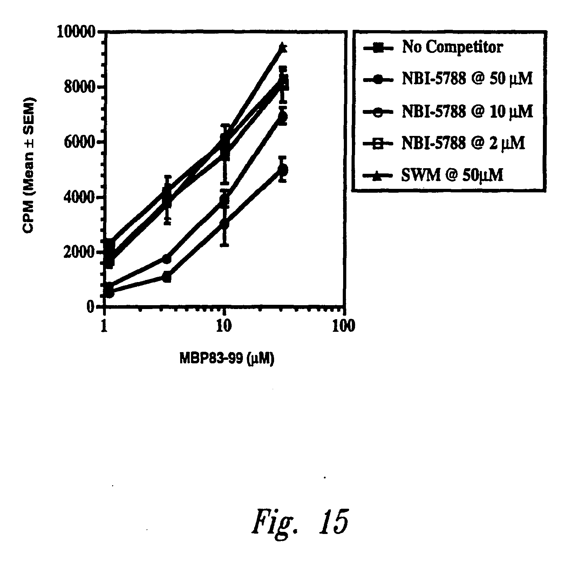

Figure 15 is a graph illustrating the ability of a peptide analog of MBP to inhibit proliferation of a Dr2a restricted human T cell clone, which is reactive to MBP. The proliferative response of the T cell clone incubated with varying concentrations of MBP (83-99) and 50 micromolar of either the peptide analog or sperm whale myoglobin, the control peptide, is depicted.

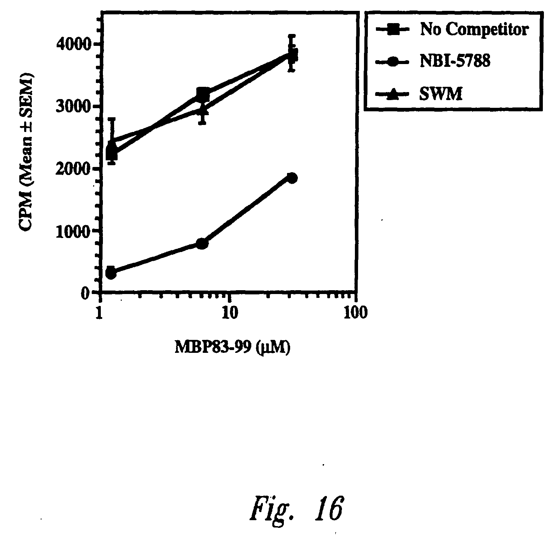

Figure 16 is a graph illustrating the ability of a peptide analog of MBP to inhibit proliferation of a Dr2b restricted human T cell clone, which is reactive to MBP. The proliferative response of the T cell clone incubated with varying concentrations of MBP (83-99) and 50 micromolar of either the peptide analog or sperm whale myoglobin, the control peptide, is depicted.

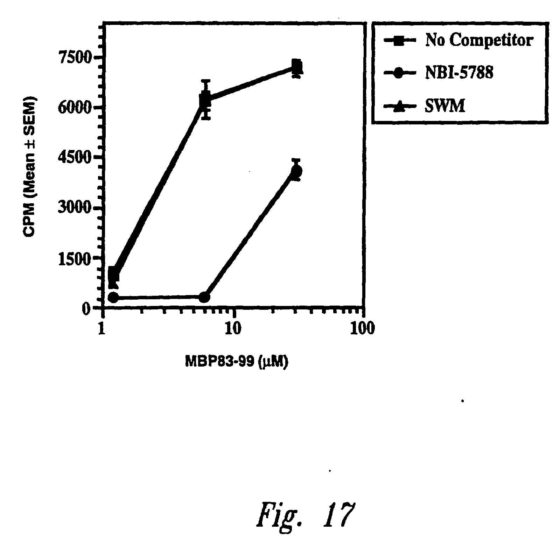

Figure 17 is a graph illustrating the ability of a peptide analog of MBP to inhibit proliferation of a Dr2b restricted human T cell clone, which is reactive to MBP. The proliferative response of the T cell clone incubated with varying concentrations of MBP (83-99) and 50 micromolar of either the peptide analog or sperm whale myoglobin, the control peptide, is depicted.

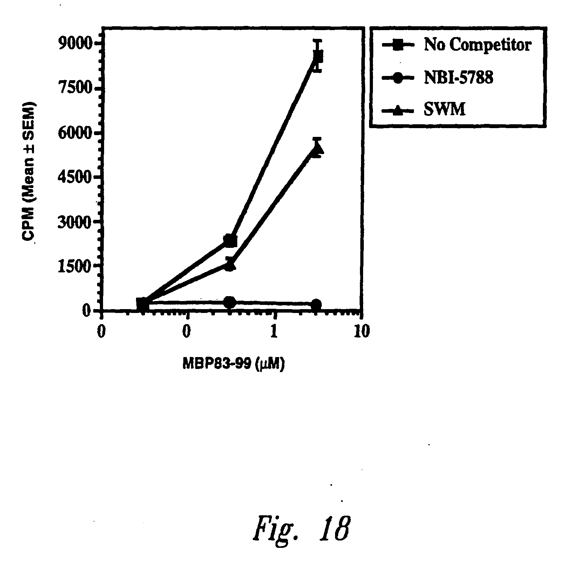

Figure 18 is a graph illustrating the ability of a peptide analog of MBP to inhibit proliferation of a Dr2b restricted human T cell clone, which is reactive to MBP. The proliferative response of the T cell clone incubated with varying concentrations of MBP (83-99) and 50 micromolar of either the peptide analog or sperm whale myoglobin, the control peptide, is depicted.

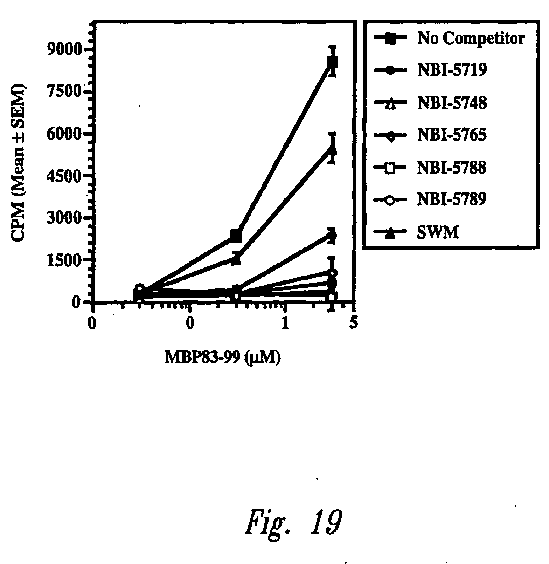

Figure 19 is a graph illustrating the ability of a peptide analog of MBP to inhibit proliferation of a Dr2b restricted human T cell clone, which is reactive to MBP. The proliferative response of the T cell clone incubated with varying concentrations of MBP (83-99) and 50 micromolar of either the peptide analog or sperm whale myoglobin, the control peptide, is depicted.

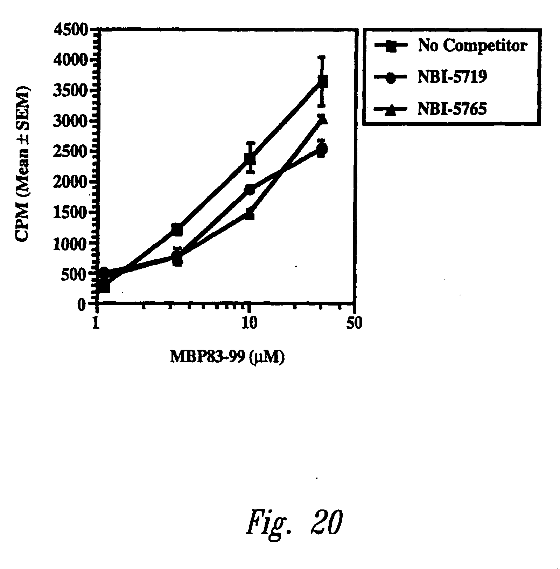

Figure 20 is a graph illustrating the ability of a peptide analog of MBP to inhibit proliferation of a Dr2b restricted human T cell clone, which is reactive to MBP. The proliferative response of the T cell clone incubated with varying concentrations of MBP (83-99) and 50 micromolar of either the peptide analog or sperm whale myoglobin, the control peptide, is depicted.

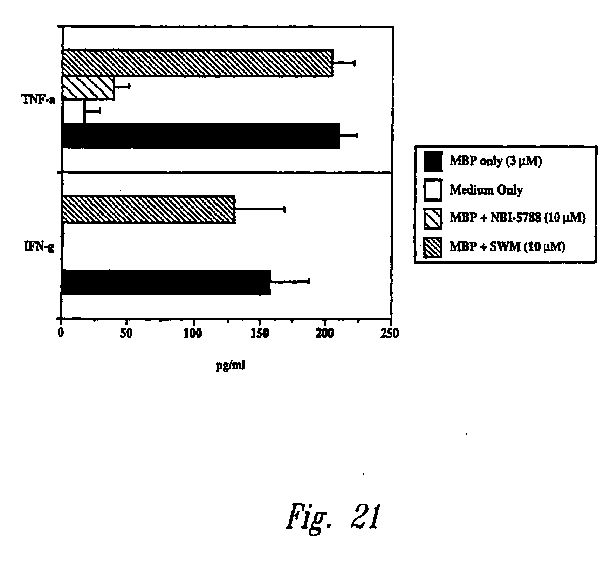

Figure 21 is a graph displaying the production of TNF-α and IFN-γ by a Dr 2b restricted human T cell clone, 5F6, which is reactive to MBP. The T cell clone was incubated in the presence of 3 µM MBP (93-99) with either 10 µM of NBI-5788 or sperm whale myoglobin or medium only. The expression level of TNF-α and IFN-γ are displayed as pg/ml.

### Detailed Description of the Invention

Prior to setting forth the invention, it may be helpful to an understanding thereof to set forth definitions of certain terms and abbreviations that will be used hereinafter.

"Human myelin basic protein" ("MBP") refers to a protein found in the cytoplasm of human oligodendroglial cells. The nucleotide sequence and predicted amino acid sequence of human MBP are presented in Figure 1 (SEQ. ID Nos. and ). Although not depicted in Figure 1, different molecular forms of human myelin basic protein generated by differential splicing or post-translational modification are also within the scope of this invention.

"Peptide analogs" of myelin basic protein are at least 7 amino acids in length and contain at least one difference in amino acid sequence between the analog and native human myelin basic protein, one of which is a difference at residue 91, 95 or 97. Unless otherwise indicated, a named amino acid refers to the L-form. An L-amino acid from the native peptide may be altered to any other one of the 20 L-amino acids commonly found in proteins, any one of the corresponding D-amino acids, rare amino acids, such as 4-hydroxyproline, and hydroxylysine, or a non-protein amino acid, such as β-alanine and homoserine. Also included with the scope of the present invention are amino acids which have been altered by chemical means such as methylation (e.g., α-methylvaline), amidation of the C-terminal amino acid by an alkylamine such as ethylamine, ethanolamine, and ethylene diamine, and acylation or methylation of an amino acid side chain function (*e.g*., acylation of the epsilon amino group of lysine).

"Residue 83," "residue 89," "residue 91," "residue 95," and "residue 97" (also called position 83, position 89, position 91, position 95, and position 97, respectively), refer to amino acids 83, 89, 91, 95, and 97 of human myelin basic protein as displayed in Figure 1 or the amino acid at a comparative position. More specifically, the numbering system for these residues relates to the amino acid position within the native human protein, regardless of the length of the peptide or the amino acid position within that peptide.

The amino acids are referred to by their standard three-letter or one-letter code. Unless otherwise specified, the L-form of the amino acid is intended. When the 1-letter code is used, a capital letter denotes the L-form and a small letter denotes the D-form. The one letter code is as follows: A, alanine; C, cysteine; D, aspartic acid; E, glutamic acid; F, phenylalanine; G, glycine; H, histidine; I, isoleucine; K, lysine; L, leucine; M, methionine; N, asparagine; P, proline; Q, glutamine; R, arginine; S, serine; T, threonine; V, valine; W, tryptophan; and Y, tyrosine.

### Peptide Analogs of Myelin Basic Protein

As noted above, the present invention provides peptide analogs comprising at least 7 amino acids selected from residues 83-99 of human myelin basic protein and including an alteration of the naturally occurring L-lysine at position 91, L-threonine at position 95, or L-arginine at position 97, to another amino acid. In one aspect, the peptide analog includes additional alteration of one to three L-amino acids at positions 86, 87, 88, 91, 95, 97, 98 and/or 99 of human myelin basic protein as long as 91 and 97 are not both altered in the same peptide analog. In another aspect, the peptide analog additionally has the N-terminal and/or C-terminal residues altered to an amino acid such that proteolysis is reduced upon administration to a patient compared to a peptide analog without these additional alterations. In a further aspect, the peptide analog of MBP comprises at least seven amino acids selected from residues 86-99 and has one of the residues at position 91, 95 or 97 altered to an amino acid not present in native MBP protein. In addition to such single alterations, one to three additional alterations of residues 86 to 99 may be made, as long as residues 91 and 97 are not altered in the same peptide analog. In yet a further aspect, the peptide analog of MBP comprises residues 83 to 99, the L-lysine at position 91 is altered to another amino acid and two to four additional amino acids selected from residues 83 to 90 and 92 to 99 are altered to another amino acid. Preferably, at least one amino acid is substituted with a charged amino acid. In addition, the N-terminal and/or C-terminal amino acids may be altered to a D-amino acid.

The peptide analogs are preferably 7 to 17 amino acids, and usually not longer than 20 amino acids. Particularly preferred peptide analogs are 14 to 17 amino acids in length. Residues 83, 89, 91, 95, and 97, which are L-glutamic acid, L-phenylalanine, L-lysine, L-threonine, and L-arginine, respectively, in the native human protein, are the key residues. Within the subject invention, analogs must have an amino acid other than L-lysine at position 91, an amino acid other than L-threonine at position 95, or an amino acid other than L-arginine at position 97.

As noted above, any amino acid alteration at position 91 is within the scope of this invention. Preferred peptide analogs include alteration of L-lysine to any one of the following amino acids: D-lysine, alanine, glycine, glutamic acid, phenylalanine, arginine, asparagine, histidine, leucine or serine. These amino acids include both conservative (similar charge, polarity, hydrophobicity, and bulkiness) and non-conservative amino acids. Although typically one might expect that only non-conservative amino acid alterations would provide a therapeutic effect, unexpectedly even conservative changes (e.g., arginine) greatly affect the function of the peptide analog as compared to the native peptide. Such diversity of substitution is further illustrated by the fact that the preferred amino acids noted above are hydrophobic and hydrophilic, charged and uncharged, polar and non-polar.

In addition, any amino acid substitution at residue 95 is also within the scope of this invention. Preferred peptide analogs contain alterations of L-threonine to any one of the following amino acids: D-threonine, alanine, glycine, isoleucine, tyrosine, glutamine, serine, lysine, glutamic acid and histidine. Other preferred alterations are to non-conservative amino acids. Particularly preferred alterations are to alanine or D-threonine.

Similarly, any amino acid alteration at position 97 is within the scope of this invention. Preferred peptide analogs include alteration of L-arginine to D-alanine, D-arginine, glycine, lysine, glutamine, glutamic acid, threonine, leucine, phenylalanine, histidine or alanine. Other preferred alterations are to non-conservative amino acids. Particularly preferred alterations are to alanine and D-arginine.

Further, any amino acid at position 83 and position 89 are within the scope of this invention. Preferred peptide analogs contain alterations of L-glutamic acid at residue 83 to any one of the following amino acids: D-alanine, L-alanine, D-glutamic acid and L-phenylalanine at position 89 to alanine, leucine, valine, isoleucine.

In addition, in certain embodiments at least one other amino acid selected from residues 86, 87, 88, 89, 95, 98, or 99 is altered. In such embodiments, if two other amino acids are changed, one is preferably selected from residues 86, 87, 88, or 89, and the other is selected from residues 98 or 99. Alternatively, up to three alterations at any positions may be made. In other embodiments, at least two to four amino acids (in addition to position 91) are altered. In such embodiments, the altered amino acids are preferably selected from positions 83, 84, 89 and 98.

With these general considerations in mind, peptide analogs within the scope of the invention have an alteration of residue 91, residue 95, or of residue 97. One set of preferred peptide analogs have double alterations. In one embodiment, residue 91 is altered as noted above, residue 87 is altered to D-valine, residue 88 to D-histidine or residue 99 to D-proline. Similarly, in another embodiment, residue 97 is altered as noted above, and either residue 87 is altered to D-valine, residue 88 to D-histidine or residue 99 to D-proline. In yet another embodiment, residue 95 is altered as noted above and residue 87 is altered to D-valine, residue 88 to D-histidine or residue 99 to D-proline.

A second set of preferred peptide analogs have three substitutions. In one embodiment, residue 91 is altered to alanine, residue 87 is altered to D-valine or residue 88 is altered to D-histidine and residue 99 is altered to D-proline. In another embodiment, residue 97 is altered to alanine, residue 88 is altered to D-histidine and residue 99 to D-proline. In yet another embodiment, residue 95 is altered to alanine, residue 88 is altered to D-histidine and residue 99 to D-proline. In still another embodiment, residue 83 is altered to D-alanine, residue 89 is altered to alanine, and residue 91 is altered to alanine.

A third set of preferred peptide analogs have four substitutions. In one embodiment, residue 83 is altered to D-alanine, residue 84 is altered to lysine, residue 89 is altered to leucine and residue 91 is altered to alanine. In another embodiment, residue 83 is altered to D-alanine, residue 84 is altered to lysine, and residues 89 and 91 are altered to alanine.

A fourth set of preferred peptide analogs have five substitutions. In one embodiment, residues 83 and 98 are altered to D-alanine, residue 84 is altered to lysine, and residues 89 and 91 are altered to alanine. In another embodiment, residues 83 and 89 are altered to D-alanine, residue 84 is altered to lysine, residue 89 is altered to leucine and residue 91 is altered to alanine.

Peptide analogs may be synthesized by standard chemistry techniques, including synthesis by automated procedure. In general, peptide analogs are prepared by solid-phase peptide synthesis methodology which involves coupling each protected amino acid residue to a resin support, preferably a 4-methyl-benzhydrylamine resin, by activation with dicyclohexylcarbodimide to yield a peptide with a C-terminal amide. Alternatively, a chloromethyl resin (Merrifield resin) may be used to yield a peptide with a free carboxylic acid at the C-terminus. Side-chain functional groups are protected as follows: benzyl for serine, threonine, glutamic acid, and aspartic acid; tosyl for histidine and arginine; 2-chlorobenzyloxycarbonyl for lysine and 2,6-dichlorobenzyl for tyrosine. Following coupling, the t-butyloxycarbonyl protecting group on the alpha amino function of the added amino acid is removed by treatment with trifluoroacetic acid followed by neutralization with di-isopropyl-ethylamine. The next protected residue is then coupled onto the free amino group, propagating the peptide chain. After the last residue has been attached, the protected peptide-resin is treated with hydrogen fluoride to cleave the peptide from the resin, as well as deprotect the side chain functional groups. Crude product can be further purified by gel filtration, HPLC, partition chromatography, or ion-exchange chromatography.

Peptide analogs within the present invention should (a) compete for the binding of native MBP peptide (e.g., 87-99 in rats; 83-99 in humans) to MHC; (b) not cause proliferation of an MBP (87-99)-reactive T cell line; and (c) inhibit induction of experimental allergic encephalomyelitis (EAE) by MBP (87-99) in rodents.

Thus, candidate peptide analogs may be screened for their ability to treat MS by (1) an assay measuring competitive binding to MHC, (2) an assay measuring a T cell proliferation, and (3) an assay assessing induction inhibition of EAE. Those analogs that inhibit binding of the native peptides, do not stimulate proliferation of MBP-reactive cell lines, and inhibit the development of EAE by native human MBP (87-99), are useful therapeutics. Although not essential, a further safety assay may be performed to demonstrate that the analog does not itself induce EAE.

Binding of peptides to MHC molecules may be assayed on whole cells. Briefly, Lewis rat spleen cells are cultured for 3 hours to allow adherent cells to stick to polystyrene petri dishes. Non-adherent cells are removed. Adherent cells, which contain cells expressing MHC class II molecules, are collected by scraping the dishes. The binding of peptide analogs to cells is measured by a fluorescence assay. In this assay, splenic adherent cells are mixed with different concentrations of peptide analogs and incubated for 1 hour at 37° in a CO₂ incubator. Following incubation, biotin-labeled MBP (87-99) is added to the culture wells. The cells are incubated for another hour and then washed three times in medium. Phycoerythrin-conjugated or fluorescein-conjugated streptavidin is added along with a fluorochrome-labeled OX-6 or OX-17 monoclonal antibody, which reacts with rat MHC Class II I-A and I-E, respectively. The cells are washed twice before analysis by flow cytometry. Fluorescence intensity is calculated by subtracting the fluorescence value obtained from cells stained with phycoerythrin-streptavidin alone (control staining) from the fluorescence value obtained from biotin-labeled MBP native peptide plus phycoerythrin-streptavidin (experimental staining). Staining without analog establishes a 100% value. Percent inhibition is calculated for each analog and expressed as IC₅₀ values. A peptide analog with an IC₅₀ value of less than 100 µM is suitable for further screenings.

Candidate peptide analogs are further tested for their property of causing or inhibiting proliferation of T cell lines. Two different assays may be used as alternatives. The first measures the ability of the analog to cause proliferation of T cells in a direct fashion. The second measures the ability of the peptide analog to inhibit proliferation of T cells induced by native MBP peptide.

In the direct proliferation assay, MBP (87-99) reactive T cell lines may be used as target cells. T cell lines are established from lymph nodes taken from rats injected with MBP (87-99). Lymph node cells are isolated and cultured for 5 to 8 days with MBP (87-99) and IL-2 as a source of T cell growth factors. Viable cells are recovered and a second round of stimulation is performed with MBP (87-99 or 83-99) and irradiated splenocytes as a source of growth factors. After 5 to 6 passages in this manner, the proliferative potential of the cell lines are determined. MBP-reactive lines are used in the proliferation assay. In this assay, T cell lines are cultured for three days with various concentrations of peptide analogs and irradiated, autologous splenocytes. After three days, 0.5-1.0 µCi of [³H]-thymidine is added for 12-16 hours. Cultures are harvested and incorporated counts determined. Mean CPM and standard error of the mean are calculated from triplicate cultures.

As an alternative to the use of T cell lines described above, draining lymph node cells from Lewis rats injected with MBP (87-99) may be used. Preferably, this assay is used in combination with the proliferation assay using T cell lines. Briefly, Lewis rats are injected subcutaneously with MBP (87-99) peptide in complete Freund's adjuvant. Nine to ten days later, draining lymph node cells are isolated and single-cell suspensions are prepared. Lymph node cells are incubated with various concentrations of peptide analogs for three days in a humidified air chamber containing 6.5% CO₂. After incubation, the cultures are pulsed with 1-2 µCi of [³H]-thymidine for 12-18 hours. Cultures are harvested on fiberglass filters and counted in a scintillation counter. Mean CPM and the standard error of the mean are calculated from data determined in triplicate cultures. Peptide analogs yielding results that are more than three standard deviations below the mean response from a comparable concentration of MBP (87-99) are considered non-stimulatory. Peptide analogs which do not stimulate proliferation at concentrations of less than or equal to 50 µM are suitable for further screenings.

The second or alternative assay is a competition assay for T cell proliferation. In this assay, antigen presenting spleen cells are first irradiated and then incubated with native MBP (87-99) peptide for 2-4 hours. These cells are then washed and further cultured with T cells reactive to MBP (87-99). Various concentrations of candidate peptide analogs are included in cultures for an additional 3 days. Following this incubation period, each culture is pulsed with 1 µCi of [³H]-thymidine for an additional 12-18 hours. Cultures are then harvested on fiberglass filters and counted as above. Mean CPM and standard error of the mean are calculated from data determined in triplicate cultures. Peptide analogs which inhibit proliferation to approximately 25% at a concentration of 50 µM or greater are suitable for further screening.

Human T cells reactive to MBP (83-99) may alternatively be used to measure the ability of the peptide analog to inhibit proliferation of T cells induced by native MBP (83-99) peptide. MBP-specific T cells may be obtained as previously described by Martin et al., *J. Immunol. 148*:1359-1366, 1992. Briefly, T cell lines are established by culture of human T cells with irradiated, DR-matched peripheral blood cells in MEM supplemented with 2 mM L-glutamine, 50 µg/ml gentamicin, penicillin and streptomycin, 100 U/ml rIL-2, and 10% human AB negative serum. Proliferation of these T cell lines is stimulated by culturing a clone with varying concentration (1.1-30 µM) of native MBP (89-99) peptide, 50 µM of the peptide analog or SWM peptide, in the presence of irradiated, DR matched peripheral blood cells, following incubation for approximately 60 hours, the cells are pulsed with ³H-thymidine for 12 hours and harvested. The amount of incorporated ³H-thymidine is measured.

As discussed in detail below, the production of cytokines may also be assessed. In particular, TNF-α and IFN-γ production are especially interesting. These pro-inflammatory cytokines are thought to play a role in the pathogenesis of the disease. Briefly, T cell clone is incubated in the presence of stimulating MBP peptide and peptide analog or control peptide (SWM) or medium only. After a 24 hour incubation, the levels of TNF-α and IFN-γ in the supernatant are determined using commercially available EIA kits (Endogen, Cambridge, MA).

Candidate peptides that compete for binding of MBP (87-99) to MHC and do not cause direct proliferation of T cell line or can inhibit proliferation by MBP (87-99), are further tested for their ability to inhibit the induction of EAE by MBP (87-99). Briefly, 500 µg of MBP (87-99) is injected as an emulsion in complete Freund's adjuvant supplemented with heat killed *Mycobacterium tuberculosis* (H37Ra). Rats are injected subcutaneously at the base of the tail with 200 µl of the emulsion. Rats are divided into two groups. Approximately 2 days prior to disease induction (usually 10 days following injection of MBP (87-99)) rats are injected intraperitoneally either with PBS or peptide analogs in PBS. Animals are monitored for clinical signs on a daily basis by an observer blind to the treatment protocol. EAE is scored on a scale of 0-4: 0, clinically normal; 1, flaccid tail paralysis; 2, hind limb weakness; 3, hind limb paralysis; 4, front and hind limbs affected. Peptide analogs injected at 5 mg/kg or less (approximately 1 mg per rat) are considered to inhibit the development of EAE if there is a 50% reduction in the mean cumulative score over seven days following onset of disease symptoms in the control group.

In addition, as a safety measure, but not essential to this invention, suitable peptide analogs may be tested for direct induction of EAE. As described in detail in Example 2, various amounts of peptide analogs are injected at the base of the tail of rats, and the rats examined daily for signs of EAE. A peptide analog which is not considered to cause EAE has a mean cumulative score of less than or equal to 1 over seven days when 1 mg (5 mg/kg) in complete Freund's adjuvant is injected.

### Treatment and Prevention of Multiple Sclerosis

As noted above, the present invention provides methods for treating and preventing multiple sclerosis by administering to the patient a therapeutically effective amount of a peptide analog of human myelin basic protein as described herein. Patients suitable for such treatment may be identified by criteria establishing a diagnosis of clinically definite MS as defined by the workshop on the diagnosis of MS (Poser et al., *Ann*. *Neurol*. *13*:227, 1983). Briefly, an individual with clinically definite MS has had two attacks and clinical evidence of either two lesions or clinical evidence of one lesion and paraclinical evidence of another, separate lesion. Definite MS may also be diagnosed by evidence of two attacks and oligoclonal bands of IgG in cerebrospinal fluid or by combination of an attack, clinical evidence of two lesions and oligoclonal band of IgG in cerebrospinal fluid. Slightly lower criteria are used for a diagnosis of clinically probable MS.

Effective treatment of multiple sclerosis may be examined in several different ways. Satisfying any of the following criteria evidences effective treatment. Three main criteria are used: EDSS (extended disability status scale), appearance of exacerbations or MRI (magnetic resonance imaging).

The EDSS is a means to grade clinical impairment due to MS (Kurtzke, *Neurology 33*:1444, 1983). Eight functional systems are evaluated for the type and severity of neurologic impairment. Briefly, prior to treatment, patients are evaluated for impairment in the following systems: pyramidal, cerebella, brainstem, sensory, bowel and bladder, visual, cerebral, and other. Follow-ups are conducted at defined intervals. The scale ranges from 0 (normal) to 10 (death due to MS). A decrease of one full step defines an effective treatment in the context of the present invention (Kurtzke, *Ann. Neurol. 36*:573-79, 1994).

Exacerbations are defined as the appearance of a new symptom that is attributable to MS and accompanied by an appropriate new neurologic abnormality (IFNB MS Study Group, *supra*). In addition, the exacerbation must last at least 24 hours and be preceded by stability or improvement for at least 30 days. Briefly, patients are given a standard neurological examination by clinicians. Exacerbations are either mild, moderate, or severe according to changes in a Neurological Rating Scale (Sipe et al., *Neurology 34*:1368, 1984). An annual exacerbation rate and proportion of exacerbation-free patients are determined. Therapy is deemed to be effective if there is a statistically significant difference in the rate or proportion of exacerbation-free patients between the treated group and the placebo group for either of these measurements. In addition, time to first exacerbation and exacerbation duration and severity may also be measured. A measure of effectiveness as therapy in this regard is a statistically significant difference in the time to first exacerbation or duration and severity in the treated group compared to control group.

MRI can be used to measure active lesions using gadolinium-DTPA-enhanced imaging (McDonald et al. *Ann. Neurol. 36*:14, 1994) or the location and extent of lesions using T₂-weighted techniques. Briefly, baseline MRIs are obtained. The same imaging plane and patient position are used for each subsequent study. Positioning and imaging sequences are chosen to maximize lesion detection and facilitate lesion tracing. The same positioning and imaging sequences are used on subsequent studies. The presence, location and extent of MS lesions are determined by radiologists. Areas of lesions are outlined and summed slice by slice for total lesion area. Three analyses may be done: evidence of new lesions, rate of appearance of active lesions, percentage change in lesion area (Paty et al., *Neurology 43*:665, 1993). Improvement due to therapy is established when there is a statistically significant improvement in an individual patient compared to baseline or in a treated group versus a placebo group.

Candidate patients for prevention may be identified by the presence of genetic factors. For example, a majority of MS patients have HLA-type DR2a and DR2b. The MS patients having genetic dispositions to MS who are suitable for treatment fall within two groups. First are patients with early disease of the relapsing remitting type. Entry criteria would include disease duration of more than one year, EDSS score of 1.0 to 3.5, exacerbation rate of more than 0.5 per year, and free of clinical exacerbations for 2 months prior to study. The second group would include people with disease progression greater than 1.0 EDSS unit/year over the past two years.

Efficacy of the peptide analog in the context of prevention is judged based on the following criteria: frequency of MBP reactive T cells determined by limiting dilution, proliferation response of MBP reactive T cell lines and clones, cytokine profiles of T cell lines and clones to MBP established from patients. Efficacy is established by decrease in frequency of reactive cells, a reduction in thymidine incorporation with altered peptide compared to native, and a reduction in TNF and IFN-α. Clinical measurements include the relapse rate in one and two year intervals, and a change in EDSS, including time to progression from baseline of 1.0 unit on the EDSS which persists for six months. On a Kaplan-Meier curve, a delay in sustained progression of disability shows efficacy. Other criteria include a change in area and volume of T2 images on MRI, and the number and volume of lesions determined by gadolinium enhanced images.

Peptide analogs of the present invention may be administered either alone, or as a pharmaceutical composition. Briefly, pharmaceutical compositions of the present invention may comprise one or more of the peptide analogs described herein, in combination with one or more pharmaceutically or physiologically acceptable carriers, diluents or excipients. Such compositions may comprise buffers such as neutral buffered saline, phosphate buffered saline and the like, carbohydrates such as glucose, mannose, sucrose or dextrans, mannitol, proteins, polypeptides or amino acids such as glycine, antioxidants, chelating agents such as EDTA or glutathione, adjuvants (*e.g*., aluminum hydroxide) and preservatives. In addition, pharmaceutical compositions of the present invention may also contain one or more additional active ingredients, such as, for example, cytokines like β-interferon.

Compositions of the present invention may be formulated for the manner of administration indicated, including for example, for oral, nasal, venous, intracranial, intraperitoneal, subcutaneous, or intramuscular administration. Within other embodiments of the invention, the compositions described herein may be administered as part of a sustained release implant. Within yet other embodiments, compositions of the present invention may be formulated as a lyophilizate, utilizing appropriate excipients which provide stability as a lyophilizate, and subsequent to rehydration.

Pharmaceutical compositions of the present invention may be administered in a manner appropriate to the disease to be treated (or prevented). The quantity and frequency of administration will be determined by such factors as the condition of the patient, and the type and severity of the patient's disease. Within particularly preferred embodiments of the invention, the peptide analog or pharmaceutical compositions described herein may be administered at a dosage ranging from 5 to 50 mg/kg, although appropriate dosages may be determined by clinical trials. Patients may be monitored for therapeutic effectiveness by MRI, EDSS, and signs of clinical exacerbation, as described above.

The following examples are offered by way of illustration and not by way of limitation.

### EXAMPLE 1

### Preparation of Peptides

The peptides were synthesized by solid phase methodology on a peptide synthesizer (Beckman model 990). Peptides with an amidated carboxyl-terminus were prepared with a p-methylbenzhydrylamine resin (MBHA resin); for peptides with a free carboxyl-terminus, a Merrifield resin coupled with the appropriately protected amino acid was used. Both resins were obtained from Bachem Fine Chemicals (Torrance, CA). Derivatized amino acids (Bachem Fine Chemicals) used in the synthesis were of the L-configuration unless specified otherwise, and the N-alpha-amino function protected exclusively with the t-butyloxycarbonyl group. Side-chain functional groups were protected as follows: benzyl for serine, threonine, glutamic acid, and aspartic acid; tosyl for histidine and arginine; 2-chlorobenzyloxycarbonyl for lysine and 2,6-dichlorobenzyl for tyrosine. Coupling of the carboxyl-terminal amino acid to the MBHA resin was carried out with dicyclohexylcarbodiimide and the subsequent amino acids were coupled with dicyclohexylcarbodiimide according to Ling et al. (*Proc. Natl. Acad. Sci. USA 81*:4302, 1984). After the last amino acid was incorporated, the t-butyoxycarbonyl protecting group was removed and the peptide-resin conjugate treated with a mixture of 14 ml hydrofluoric acid (HF), 1.4 ml anisole, and 0.28 ml methylethyl sulfide per gram of resin conjugate at -20°C for 0.5 hr and at 0°C for 0.5 hr. HF was removed in vacuum at 0°C, and the resulting peptide and resin mixture was washed twice with diethyl ether and twice with chloroform and diethyl ether alternately. The peptide was extracted five times with 2 M acetic acid, and the extract lyophilized. The lyophilized product was first purified on a column of Sephadex G-25 fine (Pharmacia-LKB, Piscataway, NJ) developed in 30% acetic acid to remove the truncated fragments and inorganic salts (Ling et al., 1984). Next, peptides were further purified by CM-32 carboxymethylcellulose cation-exchange chromatography (Ling et al., 1984). Final purification was achieved by partition chromatography on Sephadex G-25 fine (Ling et al., 1984). The synthetic product was characterized by amino acid analysis, mass spectrometric analysis, and reversed-phase HPLC.

### EXAMPLE 2

### Immunizations and EAE induction

MBP peptide and peptide analogs were dissolved in phosphate-buffered saline (PBS) and emulsified with an equal volume of incomplete Freund's adjuvant supplemented with 4 mg/ml heat-killed *Mycobacterium tuberculosis* H37Ra in oil (Difco Laboratories, Inc., Detroit, MI). Rats were immunized subcutaneously at the base of the tail with 0.1-0.2 ml containing 500 µg of peptide in the emulsion and were monitored for clinical signs daily. EAE was scored on a scale of 0-4, as follows: 0, clinically normal; 1, flaccid tail; 2, hind limb weakness; 3, hind limb paralysis; 4, front and hind limbs affected.

### EXAMPLE 3

### Long-term T cell lines

Antigen specific long-term T cell lines were derived using the method developed by Ben-Nun et al. (*Eur. J. Immunol. 11*:195, 1981). Lewis rats were injected with MBP (87-99) or MBP (83-99) as described above. Nine to ten days later draining lymph node cells were cultured (10⁷/ml) for 5-8 days in stimulation medium (Dulbecco's modified Eagle's medium supplemented with 5x10⁻⁵M 2-mercaptoethanol, 2mM L-glutamine, 1 mM sodium pyruvate, 100 µg/ml penicillin, 100 µg/ml streptomycin and 10% fetal bovine serum (Hyclone Laboratories, Logan, UT)) together with 10-20 µM of the MBP (87-99) peptide and 15 U/ml IL-2. After 5 to 8 days of culture, viable cells were collected from the interface after Ficoll-Hypaque separation and washed three times. These cells were recultured at 1 x 10⁷ cells/ml in medium with 5 x 10⁵ irradiated (3000 rad) autologous splenocytes as accessory cells and 10-20 µM of MBP (87-99). After 5 to 6 stimulation cycles, plates were screened by the ability of cells to proliferate in response to MBP (87-99). Positive lines were transferred to 24-well flat bottom plates and restimulated.

### EXAMPLE 4

### MHC binding assay

The ability of MBP peptides and peptide analogs to bind MHC was measured. An assay which characterizes the binding of peptides to MHC molecules on antigen presenting cells (APC) was employed (Mozes et al., *EMBO J*. *8*:4049, 1989; Gautam et al., *PNAS 91*:767, 1994). Spleen cells were cultured in Dulbecco's modified Eagle's medium supplemented with 10% fetal bovine serum (Hyclone Laboratories, Logan, UT) in standard polystyrene petri dishes (100 x 15 mm) in a 37°C incubator containing 6.5% CO₂ for 3 hours. Thereafter, non-adherent cells were removed, and the plates were washed three times with PBS. Adherent cells were collected using a cell scraper. The binding of MBP (87-99) analogs was measured using a fluorescence assay. Briefly, 5 x 10⁵ splenic adherent cells in staining buffer (PBS containing 0.1% bovine serum albumin) were mixed with different concentrations ranging from 0-400 µM of MBP analogs in individual wells of U-shape 96-well microculture plates and incubated for 1 hr at 37°C in a 6.5% CO₂ incubator. Following incubation, 10 µM of biotin-labeled MBP native peptide was added to culture wells for 1 h. Cells were washed three times with the staining buffer. Phycoerythrin-conjugated or fluorescein-conjugated streptavidin (Becton Dickinson, San Jose, CA) was added as a second step reagent (1 µg/well) along with 1 µg/well of fluorochrome-labeled OX-6 or OX-17 monoclonal antibody (Pharmingen, San Diego, CA), which reacts with rat MHC class II I-A or I-E, respectively. The cells were washed twice before cytofluorographic analysis on a FACScan (Becton Dickinson). Fluorescence intensity for each sample was calculated by subtracting the fluorescence obtained from OX positive cells stained with phycoerythrin-streptavidin alone (control staining) from the fluorescence obtained from OX positive cells stained with biotin-labeled MBP plus phycoerythrin-streptavidin. Percent inhibition was calculated for each analog and expressed as IC₅₀ values.

The peptide analog, h88/A91, which contains D-histidine at position 88 and alanine at position 91 competed as effectively as MBP (87-99) for MHC against MBP (87-99). At 200 µM, MBP (87-99) inhibited binding by 68.4% and h88/A91 inhibited binding by 67.64%. At 100 µM, MBP (87-89) inhibited binding by 40% and a83, A89, A91 inhibited binding by 25%.

### EXAMPLE 5

### Antigen-specific lymph node cell proliferation assay

Female Lewis rates, approximately six weeks old, were purchased from Harlan Sprague, Indianapolis, IN. MBP peptides were dissolved in phosphate-buffered saline (PBS) and emulsified with an equal volume of complete Freund's adjuvant (Difco Laboratories, Inc., Detroit, MI) supplemented with 2 mg/ml of heat-killed *Myobacterium tuberculosis* H37Ra in oil (Difco). Rats were immunized subcutaneously in the base of the tail with 0.1 ml containing 100 µg of the peptide in the emulsion. Nine to ten days following immunization, rats were sacrificed, their draining lymph node removed and a single cell suspension made. Cells were resuspended to 5 x 10⁶ cells per ml in stimulation medium containing Dulbecco's modified Eagle's medium (Gibco BRL, Gaithersburg, MD) supplemented with 2 mercaptoethanol (5 x 10⁻⁵ M), L-glutamine (2 mM), sodium pyruvate (1 mM), penicillin (100 µg/ml), streptomycin (100 µg/ml), and 1% normal rat serum.

For the assay, 100 µl of the lymph node cell suspension was added to 96-well flat-bottom wells in the presence of an equal volume of medium containing 10 µM of various peptides (including: motilin as a negative control; MBP87-99; medium only or alanine or D-amino acid substituted at position 91, 95, or 97). Cultures were then incubated at 37°C in humidified air containing 7.5% CO₂. After 3 days of incubation, 1.0 µCi of tritiated thymidine (20 Ci/mM; New England Nuclear) was added to each well and the plates reincubated for an additional 12-16 hours. The plates were then harvested with a Matrix filtermate harvester (Packard) and counted using an Automatic Direct Beta Counter (Packard). Mean cpm and the standard error of the mean were calculated from triplicate wells.

As seen in Figure 2, MBP (87-99) stimulated lymph node cells in contrast to the peptide analogs. Alanine alterations at positions 95 and 97 and D-amino acid alterations at residues 91, 95, and 97 failed to stimulate cells above the control peptide, motilin.

### EXAMPLE 6

### Antigen-specific T cell line proliferation assays

Assays for the antigen-specific proliferation assay of T cell lines were performed in 96-well flat bottom microtiter plates as described (Zamvil et al., *Nature 317*:355-358, 1985; Offner et al., *J. Immunol. 148*:1706-1711, 1992; Gold et al., *J. Immunol. 148*:1712-1717, 1992; Karin et al., *J. Exp. Med. 180*:2227-2237, 1994). T cell lines were established as described in Example 3. An initial 1:10 dilution of a 1.5 mM stock solution of MBP or the peptide analogs were added into tissue culture medium. The samples were diluted by three-fold serial dilutions (final volume 100 µl). The responding continuous T cell lines were resuspended to 4 x 10⁵ cells per ml and 50 µl aliquots added to each well (5 x 10⁴ cells per well). Approximately 1 x 10⁶ irradiated (3000R) splenocyte feeder cells were also added to each well. Cultures were then incubated at 37°C in humidified air containing 7.5% CO₂ for 3 days. Twelve to sixteen hours prior to harvesting, 0.5-1.0 µCi of [³H]-thymidine (20 Ci/mM; New England Nuclear) was added to each well and the cultures reincubated. Plates were then harvested with a Matrix filtermate harvester (Packard) and counted using an Automatic Direct Beta Counter (Packard). Mean cpm and the standard error of the mean were calculated from triplicate wells.

As can be seen in Figures 3, 4, and 5 a peptide analog with any substitution of position 91, 95, or 97 failed to stimulate proliferation of a MBP (87-99)-reactive T cell line. The effect was dramatic as even 150 µM of peptide analog was 1 to 2 logs less effective at causing proliferation.

### EXAMPLE 7

### Antagonism of T cell proliferation assay

T cell antagonism was detected in a prepulsed proliferation assay as described by De Magistris et al. (*Cell 58*:625, 1992) with minor modifications. Antigen presenting spleen cells were γ-irradiated (3000 rad) and incubated with shaking at a concentration of 10⁷ cells/well with 0.2-2.0 µM of the native peptide MBP (87-99) in stimulation medium in 10 ml tissue culture plates for 2 to 4 hours at 37°C in humidified air containing 6.5% CO₂. Spleen cells were then washed and re-cultured at a concentration of 5 x 10⁵ cells/well in U-shape 96-well microculture plates together with 5 x 10⁴ resting MBP (87-99) reactive T cells. Various concentrations of antagonist peptides, ranging from 5-150 µM, were added for an additional 72 hours. Each well was pulsed with 0.5-1 µCi of [³H]-thymidine (specific activity 10 Ci/mmol) for the final 12-16 hours. The cultures were then harvested on fiberglass filters and the proliferative response expressed as CPM±SEM.

The data presented in Figure 6 demonstrates that the double altered peptide analog, h88/A91, and the triple altered peptide analog, h88/A91/p99, significantly inhibited proliferation of a MBP reactive T cell line. The triple altered analog caused inhibition at 50 µM and higher concentration, while the double altered analog caused inhibition at 150 µM.

### EXAMPLE 8

### Treatment of 87-99 Induced EAE in Lewis Rats

Female Lewis rats, which were 6-8 weeks old, were injected with 500 µg of MBP (87-99) in CFA containing 500 µg of *Mycobacterium tuberculosis* at the base of the tail in 200 µl volume. Rats were divided in groups of 5. The control group received 0.5 ml of PBS and the treatment group received the h88/A91 peptide analog (1 mg/0.5 ml PBS) intraperitoneally, twice, on days 9 and 10 after immunization. Animals were monitored for disease symptoms on a daily basis. EAE was recorded on the following scale: 0, no symptoms; 1, tail paralysis; 2, hind limb weakness; 3, hind limb paralysis; 4, hind and front limbs affected.

Data from two different experiments was obtained as mean cumulative score of 5 animals (Figure 7). Untreated control animals went on to develop high level of disease whereas h88/A91 analog of the MBP peptide 87-99 was effective in preventing significantly the development of EAE in two experiments. Though the analog was given just before the onset of overt symptoms, it was able to arrest the development of EAE.

### EXAMPLE 9

### Induction of EAE by Peptide Analog

The ability of peptide analogs to cause EAE is assessed *in vivo*. Rats were injected with MBP (87-99) or h88/A91 peptide analog as described in Example 2. Animals were monitored daily for evidence of EAE. Rats receiving MBP (87-99) had 100% incidence (18/18 rats) of EAE with a mean maximum clinical score of 2.4 ± 0.2. In contrast, 0/12 rats receiving the peptide analog h88/A91 had EAE. Therefore, this peptide analog does not induce EAE.

### EXAMPLE 10

### Treatment of EAE With Peptide Analogs

The 91K>A peptide analog is capable of inhibiting the adoptive transfer of disease by immune T cells in the Lewis rat strain (Karin et al., 1994). Further characterization of the effects of the APL on the immune system was investigated in Lewis rates injected with HBP.

In this system, experimental allergic encephalomyelitis (EAE) was induced in twelve female Lewis rats by injection of MBP(83-99) peptide in complete Freund's adjuvant (CFA) at the base of the tail. Nine days later, rats were divided into two groups of six animals and subcutaneously injected with 13.2 mg/kg of either peptide analog or a control peptide, sperm whale myoglobin (SWM) (110-121). Animals were monitored daily for disease symptoms and scored in a blinded fashion on a nonlinear ascending scale of 0-4 with increments denoting increasing paralysis. Each individual score was averaged with group cohorts to obtain the mean clinical score. The results from one such experiment are shown in Figure 9.

As seen in Figure 9, the disease severity in those animals treated with the APL NBI-5788 (Figure 8) was about 50% reduced compared to the control group. Figure 10 shows the average disease severity of the results from three separate therapy experiments. The APL NBI-5788 significantly reduced the severity and duration of the disease in this model system. Figure 11 shows the results from treatment using another APL, NBI-5765 (Figure 8). This APL also significantly reduced the magnitude of the disease in the treated group over the control animals.

Although these results clearly demonstrate that APL inhibits the development of EAE, a murine animal model system of EAE has also been developed. The SJL/J (H-2⁵) mouse develops a chronic relapsing form of EAE in response to immunization with MBP(83-99) peptide in the presence of pertussis vaccine. The ability of the peptide analogs NBI-5719 and NBI-5765 to inhibit the disease was evaluated (see Figure 12).

Groups of 10 animals were injected intraperitoneally weekly for 4 weeks with 20 mg/kg of either a control peptide or the peptide analog. The animals were then monitored for disease over the next 2-3 months. As can be seen in Figure 12, SJL/J mice developed symptoms of EAE beginning around day 20 in the control group that lasted for approximately 3 weeks. Beginning around day 70, a relapse occurred reaching a mean clinical score of about 1. However, weekly injection with the APL NBI-5765 or NBI-5719 for four weeks not only reduced the level of the disease in the first phase, but also reduced the severity of the relapse. This is particularly striking since the animals had not been exposed to the APL for approximately one month.

### EXAMPLE 11

### Effects of Peptide Analogs on Human T Cell Proliferation

The ability of the peptide analogs NBI-5719, 5748, 5765, 5788 and 5789 to affect human T cell proliferation was assessed. A constant amount of peptide analog or the control peptide SWM (50 µM) was cultured with varying concentrations of native MBP(83-99) peptide (1.1-30 µM) in the presence of irradiated, DR matched peripheral blood cells and T cell clones derived from various MS patients. Human T cells (1 x 10⁶) were cultured with DR matched, irradiated peripheral blood cells (PBL, 5 x 10⁶) in medium containing IMDM supplemented with 3 µM MBP83-99, 2 mM L-glutamine, 50 µg/ml gentamicin penicillin/streptomycin, 100 U/ml rIL-2 and 10% human AB-negative serum. Cells were cultured for approximately 60 hours, pulsed with tritiated thymidine for 12 hours, and harvested. The amount of tritiated thymidine incorporated was measured, and the data represented as the mean plus or minus the standard error of the mean of replicate samples. Representative results are shown in Figures 13 and 14.

As seen in Figure 13, the peptide analog NBI-5788 corresponding to MBP(83-99) (83E>a, 84N>K, 89F>L, and 91K>A) inhibited the ability of a human Dr2a restricted T cell clone to respond to varying concentrations of MBP(83-99), where the irrelevant peptide (sperm whale myoglobin, SWM 110-121) had little effect on the proliferative capacity of the T cells. Figure 14 shows that all the peptides inhibited the ability of the Dr2a restricted T cells to respond to native MBP peptide in a concentration dependent fashion.

The potency of NBI-5788 was then determined by varying concentrations of the APL (2, 10, or 50 µM) in the presence of varying amounts of the native MBP(83-99) (1.1-30 µM). As seen in Figure 15, at both 10 and 50 µM, NBI-5788 significantly altered the ability of the Dr2a T cell line to respond to MBP(83-99), but no significant inhibition was seen with the irrelevant peptide SWM.

The ability of the peptide analog to inhibit the proliferative response of MBP-reactive T cells isolated from Dr2b (DrB1*1501) individuals was determined. A constant amount of NBI-5788 (50 µM) was cultured with varying concentrations of native peptide (1.1-30 µM) in the presence of irradiated, DR matched peripheral blood cells and T cell clones derived from various MS patients.

Figures 16, 17, and 18 depict results using three different T cell lines. Each T cell clone varies in the amount of thymidine incorporated in response to MBP peptide. Nevertheless, NBI-5788 inhibited the ability of the T cell clones to respond to MBP peptide in a concentration dependent fashion. The irrelevant peptide SWM had little influence on the ability of the T cells to respond to MBP peptide.

Figure 19 depicts the ability of NBI-5719, NBI-5748, NBI-5765, NBI-5788, and NBI-5789 to inhibit the MBP-dependent proliferation of the Dr2b restricted human T cell clone 5F6. As seen above with the Dr2b restricted T cells (Figure 14), the APL inhibited the MBP-dependent proliferation in a concentration dependent fashion. However, the control peptide SWM had little effect on the proliferative response.

Figure 20 depicts the ability of NBI-5719 and NBI-5765 to inhibit the MBP-dependent proliferation of the Dr4 Dw4 restricted human T cell clone MS-1. As seen above with the Dr2 restricted T cells, the APL inhibited the MBP-dependent proliferation in a concentration dependent fashion.

The ability of the peptide analog ligand to influence cytokine production was next measured. The Dr2b-restricted T cell clone 5F6 was incubated in the presence of 3 µM MBP peptide with either 10 µM of NBI-5788 or SWM or medium only. As a control, cells were cultured in the presence of medium alone. After 24 hours, supernatants were removed and the levels of tumor necrosis factor alpha (TNF-α) and interferon-γ (IFN-γ) determined using commercially available EIA kits.

As can be seen in Figure 21, MBP stimulated the production of both TNF-α and IFN-γ (approximately 200 and 160 pg/ml, respectively). However, the peptide analog ligand NBI-5788 dramatically inhibited the production of both pro-inflammatory cytokines to approximately levels achieved with medium only. The irrelevant peptide SWM had minimal effect on cytokine production. None of the peptide analogs NBI-5719, NBI-5748, NBI-5765, NBI-5788, or NBI-5789 stimulated cytokine production over background, even at concentrations of 50 µM (data not shown).

From the foregoing, it will be evident that although specific embodiments of the invention have been described herein for the purpose of illustrating the invention, various modifications may be made without deviating from the spirit and scope of the invention.

### THE PRESENT INVENTION WILL NOW BE DESCRIBED BY WAY OF REFERENCE TO THE CLAUSES BELOW:

1. A peptide analog comprising at least seven amino acids selected from residues 86 to 99 of human myelin basic protein, including residue 91, wherein the L-lysine at position 91 is altered to another amino acid, and one to three L-amino acids selected from the group consisting of valine at position 86, valine at position 87, histidine at position 88, threonine at position 95, threonine at position 98 and proline at position 99 are altered to an amino acid other than the amino acid present in the native protein at that position.
2. The peptide analog of clause 1 wherein L-lysine at position 91 is altered to a non-conservative amino acid.
3. The peptide analog of clause 1 wherein residue 91 is altered to D-lysine.
4. The peptide analog of clause 1 wherein residue 91 is altered to an amino acid selected from the group consisting of arginine, asparagine, histidine, leucine, serine, glycine, glutamic acid, phenylalanine, alanine and D-lysine.
5. The peptide analog of clause 1 wherein residue 91 is altered to alanine and residue 87 is altered to D-valine.
6. The peptide analog of clause 1 wherein residue 91 is altered to alanine and residue 88 is altered to D-histidine.
7. The peptide analog of clause 1 wherein residue 91 is altered to alanine and residue 99 is altered to D-proline.
8. The peptide analog of clause 1 wherein residue 91 is altered to alanine, residue 87 is altered to D-valine, and residue 99 is altered to D-proline.
9. The peptide analog of clause 1 wherein residue 91 is altered to alanine, residue 88 is altered to D-histidine, and residue 99 is altered to D-proline.
10. The peptide analog of clause 1 wherein residue 88 is altered to an amino acid selected from the group consisting of serine, glutamic acid, tyrosine, leucine, D-histidine, glutamine, phenylalanine and lysine.
11. A peptide analog comprising at least seven amino acids selected from residues 86 to 99 of human myelin basic protein, including residue 97, wherein the L-arginine at position 97 is altered to another amino acid and one to three L-amino acids selected from the group consisting of valine at position 86, valine at position 87, histidine at position 88, threonine at position 95, threonine at position 98 and proline at position 99 are altered to an amino acid other than the amino acid present in the native protein at that position.
12. The peptide analog of clause 11 wherein the L-arginine at position 97 is altered to a non-conservative amino acid.
13. The peptide analog of clause 11 wherein residue 97 is altered to D-arginine.
14. The peptide analog of clause 11 wherein residue 97 is altered to an amino acid selected from the group of D-alanine, D-arginine, glycine, lysine, glutamine, glutamic acid, threonine, leucine, phenylalanine, histidine and alanine.
15. The peptide analog of clause 11 wherein residue 97 is altered to alanine and residue 87 is altered to D-valine.
16. The peptide analog of clause 11 wherein residue 97 is altered to alanine and residue 88 is altered to D-histidine.
17. The peptide analog of clause 11 wherein residue 97 is altered to alanine and residue 99 is altered to D-proline.
18. The peptide analog of clause 11 wherein residue 97 is altered to alanine, residue 87 is altered to D-valine, and residue 99 is altered to D-proline.
19. The peptide analog of clause 11 wherein residue 97 is altered to alanine, residue 88 is altered to D-histidine and residue 99 is altered to D-proline.
20. The peptide analog of clause 11 wherein residue 88 is altered to an amino acid selected from the group consisting of serine, glutamic acid, tyrosine, leucine, D-histidine, glutamine, phenylalanine and lysine.
21. A peptide analog comprising at least seven amino acids selected from residues 86 to 99 of human myelin basic protein, including residue 95, wherein the L-threonine at position 95 is altered to another amino acid and one to three L-amino acids selected from the group consisting of valine at position 86, valine at position 87, histidine at position 88, threonine at position 98 and proline at position 99 are altered to an amino acid other than the amino acid present in the native protein at that position.
22. The peptide analog of clause 21 wherein the L-threonine at position 95 is altered to a non-conservative amino acid.
23. The peptide analog of clause 21 wherein residue 95 is altered to D-threonine.
24. The peptide analog of clause 21 wherein residue 95 is altered to an amino acid selected from the group consisting of alanine, D-threonine, glycine, isoleucine, tyrosine, glutamine, serine, lysine, glutamic acid and histidine.
25. The peptide analog of clause 21 wherein residue 95 is altered to alanine and residue 87 is altered to D-valine.
26. The peptide analog of clause 21 wherein residue 95 is altered to alanine and residue 88 is altered to D-histidine.
27. The peptide analog of clause 21 wherein residue 95 is altered to alanine and residue 99 is altered to D-proline.
28. The peptide analog of clause 21 wherein residue 95 is altered to alanine, residue 87 is altered to D-valine, and residue 99 is altered to D-proline.
29. The peptide analog of clause 21 wherein residue 95 is altered to alanine, residue 88 is altered to D-histidine, and residue 99 is altered to D-proline.
30. A peptide analog comprising at least seven amino acids selected from residues 86 to 99 of human myelin basic protein, including residue 91, wherein the L-lysine at position 91 is altered to another amino acid and the N-terminal amino acid and the C-terminal amino acid are altered to another amino acid, such that upon administration of the peptide analog in vivo proteolysis is reduced.
31. The peptide analog of clause 30 wherein the N-terminal and/or C-terminal amino acids are D-amino acids.
32. The peptide analog of clause 30 wherein L-lysine at position 91 is altered to a non-conservative amino acid.
33. The peptide analog of clause 30 wherein residue 91 is altered to D-lysine.
34. The peptide analog of clause 30 wherein residue 91 is altered to an amino acid selected from the group consisting of arginine, asparagine, histidine, leucine, serine, glycine, glutamic acid, phenylalanine, alanine and D-lysine.
35. A peptide analog comprising at least seven amino acids selected from residues 86 to 99 of human myelin basic protein, including residue 95, wherein the L-lysine at position 91 is altered to another amino acid and the N-terminal amino acid and the C-terminal amino acid are altered to another amino acid, such that upon administration of the peptide analog in vivo proteolysis is reduced.
36. The peptide analog of clause 35 wherein the N-terminal and/or C-terminal amino acids are D-amino acids.
37. The peptide analog of clause 35 wherein the L-threonine at position 95 is altered to a non-conservative amino acid.
38. The peptide analog of clause 35 wherein residue 95 is altered to D-threonine.
39. The peptide analog of clause 35 wherein residue 95 is altered to an amino acid selected from the group consisting of alanine, D-threonine, glycine, isoleucine, tyrosine, glutamine, serine, lysine, glutamic acid and histidine.
40. A peptide analog comprising at least seven amino acids selected from residues 86 to 99 of human myelin basic protein, including residue 97, wherein the L-lysine at position 91 is altered to another amino acid and the N-terminal amino acid and the C-terminal amino acid are altered to another amino acid, such that upon administration of the peptide analog in vivo proteolysis is reduced.
41. The peptide analog of clause 40 wherein the N-terminal and/or C-terminal amino acids are D-amino acids.
42. The peptide analog of clause 40 wherein the L-arginine at position 97 is altered to a non-conservative amino acid.
43. The peptide analog of clause 40 wherein residue 97 is altered to D-arginine.
44. The peptide analog of clause 40 wherein residue 97 is altered to an amino acid selected from the group of D-alanine, D-arginine, glycine, lysine, glutamine, glutamic acid, threonine, leucine, phenylalanine, histidine and alanine.
45. A peptide analog comprising at least seven amino acids selected from residues 86 to 99 of human myelin basic protein, including residue 91, wherein the L-lysine at position 91 is altered to another amino acid.
46. The peptide analog of clause 45 comprising seven to twelve amino acids.
47. The peptide analog of clause 45, further comprising altering one to three additional residues selected from residues 86-90, 92-96, 98 and 99 to another amino acid.
48. The peptide analog of clause 45 wherein L-lysine at position 91 is altered to a non-conservative amino acid.
49. The peptide analog of clause 45 wherein residue 91 is altered to D-lysine.
50. The peptide analog of clause 45 wherein residue 91 is altered to an amino acid selected from the group consisting of arginine, asparagine, histidine, leucine, serine, glycine, glutamic acid, phenylalanine, alanine and D-lysine.
51. A peptide analog comprising at least seven amino acids selected from residues 86 to 99 of human myelin basic protein, including residue 95, wherein the L-threonine at position 95 is altered to another amino acid.
52. The peptide analog of clause 45, further comprising altering one to three additional residues selected from residues 86-90, 92-94, and 96-99 to another amino acid.
53. The peptide analog of clause 45, further comprising altering one to three additional residues selected from residues 86-94, 96, 98 and 99 to another amino acid.
54. A peptide analog comprising at least seven amino acids selected from residues 86 to 99 of human myelin basic protein, including residue 97, wherein the L-arginine at position 97 is altered to another amino acid.
55. The peptide analog of clause 45, further comprising altering one to three additional residues selected from residues 86-90, 92-96, 98 and 99 to another amino acid.
56. A pharmaceutical composition comprising a peptide analog according to any one of clauses 1, 11, 21, 30, 35, 40, 45, 51, and 54 in combination with a physiologically acceptable carrier or diluent.
57. A method of treating multiple sclerosis, comprising:
   administering to a patient a therapeutically effective amount of a pharmaceutical composition comprising a peptide analog according to any one of clauses 1, 11, 21, 30, 35, 40, 45, 51, and 54 in combination with a physiologically acceptable carrier or diluent.
58. The method of clause 57 wherein the peptide analog comprises 14 amino acids selected from residues 86 to 90 and residue 91 is alanine, residue 88 is D-histidine and residue 99 is D-proline.
59. The method of clause 57 wherein the peptide analog comprises 14 amino acids selected from residues 86 to 90 and residue 91 is alanine, residue 87 is D-valine and residue 99 is D-proline.
60. The method of clause 57 wherein the peptide analog comprises 14 amino acids selected from residues 86 to 90 and residue 91 is alanine and residue 88 is D-histidine.
61. The method of clause 57 wherein the peptide analog comprises 14 amino acids selected from residues 86 to 90 and residue 91 is alanine and residue 87 is D-valine.
62. The method of clause 57 wherein the peptide analog comprises 14 amino acids selected from residues 86 to 90 and residue 91 is alanine and residue 99 is D-proline.
63. The method of clause 57 wherein the peptide analog comprises 14 amino acids selected from residues 86 to 90 and residue 95 is alanine, residue 87 is D-valine, and residue 99 is D-proline.
64. The method of clause 57 wherein the peptide analog comprises 14 amino acids selected from residues 86 to 90 and residue 95 is alanine, residue 88 is D-histidine and residue 99 is D-proline.
65. The method of clause 57 wherein the peptide analog comprises 14 amino acids selected from residues 86 to 90 and residue 95 is alanine and residue 88 is D-histidine.
66. The method of clause 57 wherein the peptide analog comprises 14 amino acids selected from residues 86 to 90 and residue 95 is alanine and residue 99 is D-proline.
67. The method of clause 57 wherein the peptide analog comprises 14 amino acids selected from residues 86 to 90 and residue 95 is alanine and residue 87 is D-histidine.
68. The method of clause 57 wherein the peptide analog comprises 14 amino acids selected from residues 86 to 90 and residue 97 is alanine, residue 87 is D-valine, and residue 99 is D-proline.
69. The method of clause 57 wherein the peptide analog comprises 14 amino acids selected from residues 86 to 90 and residue 97 is alanine, residue 88 is D-histidine and residue 99 is D-proline.
70. The method of clause 57 wherein the peptide analog comprises 14 amino acids selected from residues 86 to 90 and residue 97 is alanine and residue 87 is D-valine.
71. The method of clause 57 wherein the peptide analog comprises 14 amino acids selected from residues 86 to 90 and residue 97 is alanine and residue 88 is D-histidine.
72. The method of clause 57 wherein the peptide analog comprises 14 amino acids selected from residues 86 to 90 and residue 97 is alanine and residue 99 is D-proline.
73. A peptide analog comprising residues 83 to 99 of human myelin basic protein, wherein the L-lysine at position 91 is altered to another amino acid, and two to four additional L-amino acids selected from residues 83 to 90 and 92 to 99 are altered to an amino acid other than the amino acid present in the native protein at that position.
74. The peptide analog of clause 73 wherein L-lysine at position 91 is altered to alanine.
75. The peptide analog of clause 73, including a substitution of the phenylalanine at position 89 with another amino acid.
76. The peptide analog of clause 73 wherein the N-terminal amino acid and/or the C-terminal amino acid are altered to another amino acid.
77. The peptide analog of clause 76 wherein the N-terminal and/or C-terminal amino acids are altered to a D-amino acid.
78. The peptide analog of clause 77 wherein the N-terminal amino acid is residue 83 of human myelin basic protein.
79. The peptide analog of clause 73 wherein at least one of the additional L-amino acids selected from residues 83 to 90 and 92 to 99 is substituted with a charged amino acid.
80. A peptide analog comprising residues 83 to 99 of human myelin basic protein, wherein the L-glutamic acid at position 83 is altered to D-alanine, L-asparagine at position 84 is altered to L-lysine, L-phenylalanine at position 89 is altered to L-leucine, and L-lysine at position 91 is altered to L-alanine.
81. A peptide analog comprising residues 83 to 99 of human myelin basic protein, wherein L-glutamic acid at position 83 is altered to D-alanine, L-phenylalanine at position 89 is altered to L-alanine and L-lysine at position 91 is altered to L-alanine.
82. A peptide analog comprising residues 83 to 99 of human myelin basic protein, wherein the L-lysine at position 91 and the L-phenylalanine at position 89 are altered to other amino acids.
83. A pharmaceutical composition comprising a peptide analog according to any one of clauses 73 to 82 in combination with a physiologically acceptable carrier or diluent.
84. A method of treating multiple sclerosis, comprising:
   administering to a patient a therapeutically effective amount of a pharmaceutical composition according to clause 83.

## Claims

1. A peptide analogue comprising at least seven consecutive amino acids selected from residues 86 to 99 of human myelin basic protein as recited in SEQ ID NO:3, including residue 91, wherein the L-lysine at position 91 is altered to another amino acid, and the N-terminal amino acid and/or the C-terminal amino acid are altered to another amino acid, such that upon administration of the peptide analogue *in vivo* proteolysis is reduced.

2. The peptide analogue of claim 1, wherein the N-terminal or the C-terminaJ amino acid is a D-amino acid.

3. The peptide analogue of claim 1 wherein L-lysine at position 91 is altered to a non-conservative amino acid.

4. The peptide analogue of claim 1 wherein residue 91 is altered to alanine.

5. The peptide analogue of claim 1 wherein residue 91 is altered to an amino acid selected from the group consisting of arginine, asparagine, histidine, leucine, serine, glycine, glutamic acid, phenylalanine, alanine and D-lysine.

6. A peptide analogue comprising at least seven consecutive amino acids selected from residues 86 to 99 of human myelin basic protein, wherein the L-lysine at position 91 is altered to another amino acid and wherein said analogue competes for the binding of native human MBP peptide residues 83-99 to MHC and does not cause proliferation of a native MBP reactive T cell line.

7. The peptide analogue of claim 6 comprising seven to twelve amino acids.

8. The peptide analogue of claim 6 wherein L-lysine at position 91 is altered to a non-conservative amino acid.

9. The peptide analogue of claim 6 wherein residue 91 is altered to alanine.

10. The peptide analogue of claim 6 wherein residue 91 is altered to an amino acid selected from the group consisting of arginine, asparagine, histidine, leucine, serine, glycine, glutamic acid, phenylalanine, alanine and D-lysine.

11. A peptide analogue comprising at least seven consecutive amino acids selected from residues 86-99 of human myelin basic protein, including residue 91, wherein the L-lysine at position 91 is altered to another amino acid, and further comprising altering one to three additional residues selected from residues 86-90, 92-96, 98 and 99 to another amino acid.

12. A composition comprising a peptide analogue according to any one of claims 1, 6, and 11 in combination with a physiologically acceptable carrier or diluent.

13. A peptide analogue comprising at least seven consecutive amino acids selected from residues 83-99 of human myelin basic protein, wherein the L-lysine at position 91 is altered to another amino acid, and further comprising altering two to four additional residues selected from residues 83 to 90 and 92 to 99 to another amino acid.

14. The analogue of claim 13, wherein position 91 is altered to alanine.

15. The analogue of claim 13 or 14, wherein the N-terminal or the C-terminal amino acid is a D-amino acid.

16. The peptide analog comprising 83 to 99 of human myelin basic protein of Figure 1, wherein the L-lysine at position 91 is altered to another amino acid, wherein the L-glutamic acid at position 83 is altered to another amino acid, wherein the L-asparagine at position 84 is altered to another amino acid, and wherein the L-phenylalanine at position 89 is altered to another amino acid.

17. The peptide analog of claim 16, wherein L-glutamic acid at position 83 is altered to D-alanine, wherein L-asparagine at position 84 is altered to lysine, wherein L-phenylalanine at position 89 is altered to leucine, and wherein L-lysine at position 91 is altered to alanine.
